(19) **Europäisches Patentamt**
**European Patent Office**
**Office européen des brevets**

(11) **EP 1 566 372 B1**

(12) **EUROPEAN PATENT SPECIFICATION**

(45) Date of publication and mention
of the grant of the patent:
**10.01.2018 Bulletin 2018/02**

(51) Int Cl.:
***C07C 29/149*** *(2006.01)*    ***C07C 29/17*** *(2006.01)*
***C07C 209/72*** *(2006.01)*    ***C07C 33/20*** *(2006.01)*
***C07C 31/20*** *(2006.01)*

(21) Application number: **03811941.8**

(22) Date of filing: **27.11.2003**

(86) International application number:
**PCT/JP2003/015134**

(87) International publication number:
**WO 2004/048297 (10.06.2004 Gazette 2004/24)**

(54) **METHOD OF HYDROGENATION REACTION**

VERFAHREN FÜR EINE HYDRIERUNGSREAKTION

PROCEDE DE REACTION D'HYDROGENATION

(84) Designated Contracting States:
**DE**

(30) Priority: **27.11.2002 JP 2002344520**

(43) Date of publication of application:
**24.08.2005 Bulletin 2005/34**

(73) Proprietor: **NEW JAPAN CHEMICAL CO., LTD.**
**Kyoto-shi, Kyoto 612-8224 (JP)**

(72) Inventors:
• **FUJITANI, Kango**
**Uji-shi, Kyoto 611-0033 (JP)**
• **HAYASHI, Yutaka**
**Joyo-shi, Kyoto 610-0121 (JP)**

(74) Representative: **Held, Stephan et al**
**Meissner Bolte Patentanwälte**
**Rechtsanwälte Partnerschaft mbB**
**Widenmayerstraße 47**
**80538 München (DE)**

(56) References cited:
**EP-A2- 0 300 346**    **EP-A2- 0 300 347**
**WO-A1-98/00383**    **JP-A- 2 000 738**
**JP-A- 2 042 035**    **JP-A- 10 045 646**
**JP-A- 2001 089 403**    **US-A- 3 334 149**
**US-A- 4 102 778**    **US-A- 4 649 226**
**US-A- 4 942 266**    **US-A- 4 982 020**
**US-A- 5 093 535**    **US-A- 5 233 099**
**US-A- 5 386 066**

• **Anonymous: "Tablet hardness testing - Wikipedia, the free encyclopedia", , 6 July 2015 (2015-07-06), XP055205247, Retrieved from the Internet: URL:https://en.wikipedia.org/wiki/Tablet_hardness_testing [retrieved on 2015-07-29]**

Remarks:
The file contains technical information submitted after the application was filed and not included in this specification

**Description**

TECHNICAL FIELD

The present invention relates to heterogeneous catalytic hydrogenation process.

BACKGROUND OF THE INVENTION

Heterogeneous catalytic hydrogenation is a complicated reaction in which three phases of gas-liquid-solid surface are involved. In the past, there is no technique that discloses what kind of gas-liquid flow state can give high productivity.

A document, for example, discloses a heavy oil direct desulfurization apparatus used under trickle flow condition (see "Chemical Engineering" volume 34, No. 12, page 1260 (1970)). This document discloses that residual oil desulfurization in a fixed bed has many unsolved problems of chemical engineering, and in particular the flow state and liquid mixing has a great influence on scaling up. Moreover, the document describes the necessity of determining the solubility of hydrogen gas, and of investigating the relationship between catalytic activity and pore distribution because it is a liquid phase reaction and sulfur compounds are large molecules. In this document, holdup is chemically investigated with respect to starting materials with high sulfur content. However, hydrogenation of organic compounds containing ester group and carbon-carbon double bond is not described.

Other documents disclose that, in the production of saturated alcohols by carrying out ester reduction of a starting material composed of fats and oils or fatty acid esters, a low sulfur content starting material, obtained beforehand by bringing a nickel metal- or a copper metal-containing catalyst and hydrogen gas into contact with said starting material to thereby reduce the sulfur content in the fats and oils or fatty acid esters to 0.6 ppm or less, achieves a comparable reduction reactivity, compared with a starting material having the same sulfur content obtained by distilling the starting material (Japanese Patent No. 2544552 (claims, paragraphs 0023 to 0025), Japanese Patent No. 2846450 (claims), and Japanese Patent No. 2934073 (claims, paragraph 0017)).

In these Japanese Patents, however, the hydrogenation reaction used is a conventional process employing a liquid phase suspension bed or fixed bed reaction method, and thus these patents merely disclose hydrogenation of a decreased sulfur content starting material to the corresponding alcohol in the presence of a copper-based ester reduction catalyst according to a conventional process.

Heterogeneous catalytic hydrogenation is a complicated reaction in which three phases of gas-liquid-solid surface are involved. In the past, there is no technique that discloses what kind of gas-liquid flow state gives high productivity.

A prior technique developed by the applicant includes a method for producing an unsaturated alcohol by hydrogenating an unsaturated aldehyde, an unsaturated fatty acid, or an unsaturated fatty acid ester using a zinc-chromium-based or a zinc-chromium-aluminum-based composite metal oxide catalyst, in which copper content and nickel content in said composite metal oxide catalyst are decreased to a predetermined amount or less (Japanese Unexamined Patent Publication No. 2001-89403 (see Claims 1 and 2, Paragraphs 0022 and 0023)). In this method, it is preferable for the catalyst of specific composition to have a crushing strength of 20 to 500 kg/cm$^3$, a transverse crushing strength of 2 to 10 kg/cm, and a bulk specific gravity of 1.0 to 1.8. In this method, only the importance of the copper and nickel contents of the catalyst, and the crushing strength thereof are noted, and there is no mention of the gas-liquid flow state in the reaction system.

An object of the present invention is to provide a heterogeneous catalytic hydrogenation process capable of being applied to the hydrogenation of an unsaturated fatty acid alkyl ester to an unsaturated alcohol and achieving high productivity.

DISCLOSURE OF THE INVENTION

The inventors of the present invention carried out assiduous studies to attain the above object. As a result, the inventors discovered that high productivity is attained in hydrogenating an unsaturated fatty acid alkyl ester under a trickle flow condition using a reaction tower comprising a tubular reactor charged with a solid catalyst in which both hydrogen gas and a liquid-phase substance to be hydrogenated are made to flow down from the upper part, when the dynamic liquid phase holdup amount per unit catalyst exterior surface area is set at $0.005 \times 10^{-3}$ to $0.14 \times 10^{-3}$ m$^3$/m$^2$ and a solid catalyst having a minimum catalyst strength per catalyst of 1.0 kg to 4.0 kg is used.

The present invention was accomplished based on this finding and further study, and provides a heterogeneous catalytic hydrogenation process as described below.

   Item 1. A heterogeneous catalytic hydrogenation process comprising hydrogenating a substance to be hydrogenated under trickle flow condition using a reaction tower charged with a solid catalyst in which hydrogen gas and a liquid phase comprising the substance to be hydrogenated are made to flow co-currently downward from above, wherein dynamic liquid phase holdup amount per unit catalyst exterior surface area of the catalyst charged in the reaction tower is $0.005 \times 10^{-3}$ to $0.14 \times 10^{-3}$ m$^3$/m$^2$ and the catalyst has a catalyst strength of 1.0 Kg to 4.0 kg, as determined by the following formula

$$\texttt{catalyst strength = A-2}\sigma$$

wherein A represents a mean value of minimum crushing strengths of 100 catalyst samples measured according to the method described in "Test method for crushing strength" of JIS Z-8841-1993" and $\sigma$ represents a standard deviation of the minimum crushing strengths the total content of chlorine atom and sulfur atom contained in the substance to be hydrogenated that is subjected to the hydrogenation is 5 ppm or less, the solid catalyst is a zinc-based catalyst and the hydrogenation reaction is reduction of an unsaturated fatty acid alkyl ester to an unsaturated alcohol.

Item 2. The heterogeneous catalytic hydrogenation process according to Item 1, wherein the hydrogenation is reduction of a C16-C22 unsaturated fatty acid ester to a C16-C22 unsaturated alcohol.

Item 3. The heterogeneous catalytic hydrogenation process according to any one of Items 1 to 2, wherein the total content of chlorine atom and sulfur atom in the substance to be hydrogenated that is subjected to the hydrogenation is 3 ppm or less.

Item 4. The heterogeneous catalytic hydrogenation process eccording to Item 1, wherein the zinc-based catalyst is zinc-chromium oxide, zinc-aluminium oxide, zinc-aluminum-chromium oxide, zinc-chromium-manganese oxide, zinc-iron oxide or zinc-iron-aluminum oxide.

Item 5. The heterogeneous catalytic hydrogenation process according to any one of Items 1 to 4, wherein the solid catalyst has a shape selected from the group consisting of a cylinder, a hollow cylinder, a trilobal-shaped rod, a tetralobal-shaped rod and a spherical shape, and has a minimum length of 1 to 10 mm.

Item 6. The heterogeneous catalytic hydrogenation process according to any one of Items 1 to 5, wherein the dissolved hydrogen concentration in the liquid phase present in the reaction tower is 0.01 to 5.0 kmol/m$^3$.

Item 7. The heterogeneous catalytic hydrogenation process according to Item 1, wherein the dissolved hydrogen concentration in the liquid phase is maintained within 10 to 100% of the saturated hydrogen concentration in the liquid phase, at a point 1 m below the highest point of the catalyst layer in the reaction tower.

Item 8. The heterogeneous catalytic hydrogenation process according to Claim 1, wherein the hydrogenation reaction is

hydrogenation of (1) an unsaturated carboxylic acid ester to an unsaturated alcohol; and the dissolved hydrogen concentration in the liquid phase is maintained within 50 to 100% of the saturated hydrogen concentration in the liquid phase, at a point 1 m below the highest point of the catalyst layer in the reaction tower.

BRIEF DESCRIPTION OF THE DRAWINGS

Fig. 1 is a schematic view showing trickle flow condition in the present invention.
Fig. 2 is a plan view of a solid catalyst having a shape of trilobal-shaped rod, in which R is its minimum length.
Fig. 3 is a plane view of a solid catalyst having a shape of tetralobal-shaped rod, in which L is its minimum length.
Fig. 4 is a schematic view outlining the reactor used in each example and comparative example.

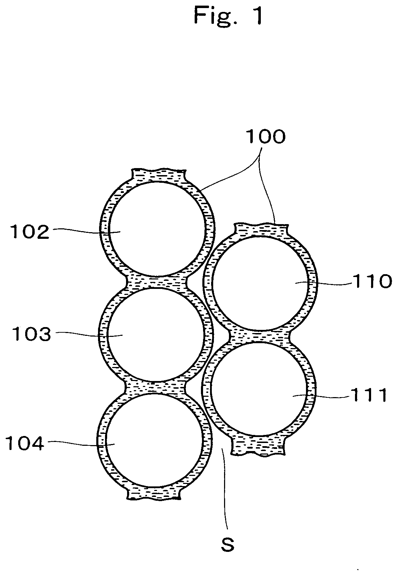

Reference numerals used in Figs. 1 to 4 denote the following:

| | |
|---|---|
| 100 | liquid phase |
| 102 | solid catalyst |
| 103 | solid catalyst |
| 104 | solid catalyst |
| 110 | solid catalyst |
| 111 | solid catalyst |
| S | space |
| a | piping for hydrogen gas introduction |
| b | piping for recycling hydrogen gas |

c     piping for removing hydrogenation products

1     reactor

2     reactor

3     reactor

4     reactor

5     high-pressure gas-liquid separator

6     low-pressure gas-liquid separator

7     high-pressure gas-liquid separator

8     high-pressure gas-liquid separator

9     high-pressure gas-liquid separator

10    heat exchanger

11    solid catalyst charged layer

12    solid catalyst charged layer

13    solid catalyst charged layer

14    solid catalyst charged layer

15    heat exchanger

16    hydrogen gas circulator

17    hydrogen gas preheater

18    starting material preheated

20    hydrogen gas compressor

61    pressure gauge

## DETAILED DESCRIPTION OF THE INVENTION

Substance to be hydrogenated and hydrogenation product

The substance to be hydrogenated used as a starting material in the hydrogenation reaction of the invention is an unsaturated fatty acids alkyl esters

The hydrogenation reaction of the invention is reduction of unsaturated fatty acid alkyl esters to unsaturated alcohols.

Hereinafter, starting materials and products obtained by hydrogenating (reducing) the starting materials according to the present invention will be described in detail. In the following description, the term "lower alkyl" is intended to mean an alkyl group having 1 to 4 carbon atoms, unless otherwise specified.

<Saturated or unsaturated fatty acids, and triglycerides and lower alkyl esters thereof>

Disclosed hereinafter is a process which can be used for producing a saturated alcohol having 8 to 22 carbon atoms from a starting material selected from the group consisting of saturated or unsaturated fatty acids, triglycerides thereof and lower alkyl esters thereof.

The starting materials for producing saturated alcohols having 8 to 22 carbon atoms include at least one member selected from triglycerides of saturated or unsaturated fatty acids having 8 to 22 carbon atoms (fats and oils), such as coconut oil, palm kernel oil, palm oil, olive oil, soybean oil, low erucic rapeseed oil, high erucic rapeseed oil, safflower oil, corn oil, cotton seed oil, sunflower oil, rice bran oil, linseed oil, etc.; used oils such as soybean oil, rapeseed oil, safflower oil, corn oil, cotton seed oil, sunflower oil, olive oil, rice bran oil, etc. used for deep-frying; beef tallow, lard, chicken fat, fish oil, etc.; saturated or unsaturated fatty acids having 8 to 22 carbon atoms derived from these fats and oils, and lower alkyl esters of said saturated or unsaturated fatty acids having 8 to 22 carbon atoms.

Among these, lower alkyl esters, in particular methyl esters, of said saturated or unsaturated fatty acids are preferred as a starting material for the reduction reaction. Before the reduction reaction, fatty acid lower alkyl esters (particularly methyl ester), fatty acids, etc. may be subjected to distillation or separation by cooling and solidification to thereby concentrate and separate a particular component having a specific number of carbon atoms, which is then used as the starting material for the reduction reaction.

To produce a saturated alcohol having 8 to 22 carbon atoms from a starting material selected from the group consisting of the above-mentioned saturated or unsaturated fatty acids and triglycerides and lower alkyl esters thereof, it is preferable to carry out the heterogeneous catalytic hydrogenation process of the present invention using a copper-based solid catalyst, which will be described later.

<Unsaturated fatty acid alkyl esters> (according to the invention)

The process of the invention can be used for producing an unsaturated alcohol having 16 to 22 carbon atoms from a starting material selected from the group consisting of unsaturated fatty acids lower alkyl ester.

The unsaturated alcohol to be produced includes unsaturated alcohols having 16 to 22 carbon atoms that have at least one (particularly 1 to 3) unsaturated bonds per molecule. More specifically, elaidyl alcohol, oleyl alcohol, linoleyl alcohol, linolenyl alcohol, etc., may be mentioned.

The starting material for producing the above-mentioned unsaturated alcohols includes lower alkyl esters of unsaturated fatty acids having 16 to 22 carbon atoms. Among these, the lower alkyl esters, in particular methyl esters, of said unsaturated fatty acids are preferred as a starting material for the reduction reaction.

Among the starting materials for the reduction reaction, the above-mentioned unsaturated fatty acid alkyl esters can be obtained by esterifying the unsaturated fatty acids thus obtained with a lower alcohol (for example, a $C_1$-$C_4$ alcohol, such as methyl alcohol), and can also be obtained by transesterification of vegetable fats and oils with a $C_1$-$C_4$ alcohol such as methyl alcohol.

In general, the starting material selected from the group consisting of unsaturated fatty acid alkyl esters is preferably subjected to distillation or if desired to separation by cooling and solidification, so as to convert it to a reduction reaction starting material having an iodine value of 40 to 200.

Waxy esters of unsaturated fatty acid and unsaturated alcohol (esters of a long-chain unsaturated fatty acid and a long-chain unsaturated alcohol, i.e., wax esters), produced during the reduction reaction or during distillation of the reduction reaction product, can also be used as a starting material for the reduction reaction.

For producing unsaturated alcohols having 16 to 22 carbon atoms from the above-mentioned starting materials, the heterogeneous catalytic hydrogenation of the present invention is carried out using a zinc-based solid catalyst, which will be described later.

<Aliphatic dicarboxylic acid dialkyl esters, hydroxyl-alkanoic acids and lower alkyl esters and lactones thereof>

Disclosed is further a process which can be used for producing aliphatic diols having 3 to 22 carbon atoms from aliphatic dicarboxylic acid dialkyl esters, hydroxyl-alkanoic acids and lower alkyl esters and lactones thereof, etc.

Examples of aliphatic diols having 3 to 22 carbon atoms that can be produced include, for example, 1,3-propanediol, 1,4-butanediol, 1,5-pentanediol, 1,6-hexanediol, 1,7-heptanediol, 1,8-octanediol, 1,9-nonanediol, 1,10-decanediol, 1,12-dodecanediol, 1,13-tridecanediol, etc.

Examples of the starting materials for producing such aliphatic diols include (a) di(lower alkyl) esters of aliphatic dicarboxylic acids having 3 to 22 carbon atoms which may have one or more (particularly 1 to 2) double bonds, (b) lower alkyl esters of aliphatic monocarboxylic acids having 3 to 22 carbon atoms and having one hydroxyl group, (c) oligo esters produced by the reaction of a $C_3$-$C_{22}$ aliphatic dicarboxylic acid which may have one or more (particularly 1 to 2) double bonds with a $C_3$-$C_{22}$ aliphatic monocarboxylic acid having one hydroxyl group or its hydrogenation product, i.e., $C_3$-$C_{22}$ aliphatic diols, (d) lactones obtained from a $C_3$-$C_{22}$ aliphatic monocarboxylic acids having one hydroxyl group, etc. Examples of lower alkyl group include alkyl groups having 1 to 4 carbon atoms, such as methyl group, ethyl group, n-propyl group, n-butyl group, etc., among which methyl group is particularly preferred.

In producing 1,3-propanediol, for example, starting materials for producing 1,3-propanediol include 1-hydroxy-propanoic acid lower alkyl esters (e.g., methyl ester), which are adducts of water and the double bond of an acrylic acid lower alkyl ester (e.g., methyl ester). Also usable are condensation products (in particular, oligo esters) of 1-hydroxy-propanoic acid lower alkyl ester involving the removal of methyl alcohol. Moreover, condensation products (in particular, oligo esters) of 1-hydroxypropanoic acid and its reduction product 1,3-propanediol can also be used.

In producing 1,4-butanediol, starting materials for producing 1,4-butanediol include, for example, maleic acid di(lower alkyl) esters, succinic acid di(lower alkyl) esters, γ-butyrolactone, etc.

In producing alkyl($C_1$-$C_4$)-substituted-1,4-butanediols, starting materials include, for example, alkyl ($C_1$-$C_4$)-substituted-maleic acid dialkyl($C_1$-$C_4$) esters, alkyl ($C_1$-$C_4$)-substituted-succinic acid dialkyl($C_1$-$C_4$) esters, alkyl ($C_1$-$C_4$)-substituted-γ-butyrolactone. The above-mentioned alkyl groups include linear or branched alkyl groups having 1 to 4 carbon atoms, such as methyl, ethyl, n-propyl, iso-propyl, n-butyl, etc., and its substitution position is not particularly limited.

In producing 1,6-hexanediol, starting materials include, for example, oligomers obtained by subjecting adipic acid di(lower alkyl) esters, ε-caprolactone, 1-hydroxy-caproic acid and/or 1-hydroxy-caproic acid lower alkyl ester to dehydrative ester condensation and/or transesterification; and also include oligomers of 1,6-hexanediol and 1-hydroxy-caproic acid and/or 1-hydroxy-caproic acid lower alkyl esters, oligo esters of 1,6-hexanediol and adipic acid, etc.

For producing an aliphatic diol from the above-mentioned starting material, it is preferable to carry out the heterogeneous catalytic hydrogenation process of the invention using a copper-based solid catalyst, which will be described later.

<Alicyclic dicarboxylic acid dialkyl esters>

Disclosed is further a process which can be used to produce alicyclic diols having 8 to 12 carbon atoms from alicyclic dicarboxylic acid di($C_1$-$C_{10}$)alkyl esters.

Examples of alicyclic diols having 8 to 12 carbon atoms to be produced include, for example, 1,2-cyclohexane-dimethanol, 1,3-cyclohexane-dimethanol, 1,4-cyclohexane-dimethanol, 1,5-decalin-dimethanol, 2,6-decalin-dimethanol, etc.

Starting materials for producing such alicyclic diols include alicyclic dicarboxylic acid di($C_1$-$C_{10}$)alkyl esters which may have one or more (particularly 1 to 2) double bonds per molecule. The alkyl groups include linear or branched aliphatic groups, such as methyl, ethyl, n-propyl, iso-propyl, n-butyl, sec-butyl, iso-butyl, n-pentyl, n-hexyl, n-heptyl, n-octyl, etc., alicyclic groups, such as cyclopentyl, cyclohexyl, 2-methylcyclohexyl, 3-methylcyclohexyl, 4-methylcyclohexyl, etc.

Starting materials for producing 1,2-cyclohexane-dimethanol include $\Delta^4$-tetrahydrophthalic acid di($C_1$-$C_{10}$)alkyl esters, 1,2-hexahydrophthalic acid di($C_1$-$C_{10}$)alkyl esters, etc. Starting materials for producing 1,4-cyclohexane-dimethanol include, for example, 1,4-hexahydrophthalic acid di($C_1$-$C_{10}$)alkyl esters.

Starting materials for producing 1,5-decalin-dimethanol or 2,6-decalin-dimethanol include, for example, 1,5-decalin-dicarboxylic acid di($C_1$-$C_4$)alkyl esters and 2,6-decalin-dicalboxylic acid di($C_1$-$C_4$)alkyl esters.

In general, for producing alicyclic diols using the above-mentioned alicyclic dicarboxylic acid dialkyl esters as starting materials, it is preferable to use a copper-based solid catalyst, which will be described later, for carrying out the heterogeneous catalytic hydrogenation process of the present invention.

<Dicarboxylic acid anhydrides>

Disclosed is further a process which can be used for producing γ-butyrolactone-based compounds which are intramoleculer esterification reaction products from dicarboxylic acid anhydrides. More specifically, γ-butyrolactone-based compounds include γ-butyrolactone, phthalide, hexahydrophthalide, etc.

Starting materials for producing γ-butyrolactone-based compounds include maleic anhydride, succinic anhydride, phthalic anhydride, $\Delta^4$-tetrahydrophthalic anhydride, hexahydrophthalic anhydride, etc.

In general, for producing the above-mentioned γ-butyrolactone-based compounds using dicarboxylic acid anhydrides, it is preferable to use a nickel-based solid catalyst or a noble-metal-based solid catalyst, which will be described later, for carrying out the heterogeneous catalytic hydrogenation process of the present invention.

<Hydrogenation of double bonds>

Disclosed are further to reactions for hydrogenating carbon-carbon double bonds.

For example, hydrogenated fats and oils can be prepared from fats and oils that are glycerides of unsaturated fatty acids, and is applicable to a process for hydrogenating any carbon-carbon double bonds remaining in a very small amount in saturated aliphatic alcohols having 8 to 22 carbon atoms, to reduce the iodine value thereof and to thereby produce a high-quality saturated alcohols. In general, in the above-mentioned process, it is preferable to use a nickel-based solid catalyst for carrying out the heterogeneous catalytic hydrogenation process of the present invention.

Disclosed is further a process in which an unsaturated alcohol ($C_{16}$-$C_{22}$, particularly $C_{18}$) that contains conjugated double bonds within the molecule is used as starting material, and which comprises hydrogenating conjugated diene moiety in said unsaturated alcohol to monoene moiety to thereby produce unsaturated alcohols containing no conjugated diene compound. The unsaturated alcohol thus obtained has excellent stability over time and heat resistance. In this case, it is usually preferable to use a copper-based solid catalyst for carrying out the heterogeneous catalytic hydrogenation process.

Disclosed is further a process for producing succinic anhydride by hydrogenating maleic anhydride, a process for producing hexahydrophthalic anhydride by hydrogenating $\Delta^4$-tetrahydrophthalic anhydride, a process for producing hexahydrophthalic acid dialkyl (linear or branched, $C_1$-$C_{13}$) esters by hydrogenating $\Delta^4$-tetrahydrophthalic acid dialkyl (linear or branched, $C_1$-$C_{13}$) esters, etc. In this case, it is usually preferable to use a nickel-based solid catalyst or a noble-metal-based solid catalyst, which will be described later.

<Nuclear hydrogenation of aromatic compounds or heterocyclic compounds>

Disclosed is further the hydrogenation of aromatic compounds. Aromatic compounds as starting materials include compounds containing one or more benzene or naphthalene rings.

{Production of cyclohexane dicarboxylic acid di($C_1$-$C_{13}$)alkyl esters}

Starting materials for producing cyclohexane dicarboxylic acid di($C_1$-$C_{13}$)alkyl esters include di ($C_1$-$C_{13}$) alkyl terephtalates, di ($C_1$-$C_{13}$) alkyl isophtalate, di ($C_1$-$C_{13}$)alkyl terephtalates, etc.

Examples of $C_1$-$C_{13}$ alkyl groups include linear or branched aliphatic groups, such as methyl, ethyl, n-propyl, iso-propyl, n-butyl, sec-butyl, iso-butyl, n-pentyl, n-hexyl, n-heptyl, and n-octyl; and alicyclic groups, such as cyclopentyl, cyclohexyl, 2-methylcyclohexyl, 3-methylcyclohexyl, 4-methylcyclohexyl, etc. {Production of compounds that have a cyclohexane ring or saturated fused ring}

In producing cyclohexane, methylcyclohexane, dimethylcyclohexane, ethylcyclohexane, trimethylcyclohexane, methylethylcyclohexane, decalin, compounds obtained by nuclear hydrogenation of petroleum $C_8$ distillate aromatic hydrocarbons (for example, tradename "RIKASOLV 800", manufactured by New Japan Chemical Co., Ltd., compounds obtained by nuclear hydrogenation of petroleum $C_9$ distillate aromatic hydrocarbons (for example, tradename "RIKASOLV 900", manufactured by New Japan Chemical Co., Ltd., compounds obtained by nuclear hydrogenation of petroleum $C_{10}$ distillate aromatic hydrocarbons, etc., examples of starting materials include benzene, toluene, xylene, ethylbenzene, trimethylbenzene, methylethylbenzene, naphthalene, petroleum $C_8$-distillate aromatic hydrocarbons, petroleum $C_9$-distillate aromatic hydrocarbons, petroleum $C_{10}$-distillate aromatic hydrocarbons, etc.

{Production of alicyclic monool}

In producing cyclohexanol, methylcyclohexanol, dimethylcyclohexanol, ethylcyclohexanol, t-butylcyclohexanol, di(t-butyl)cyclohexanol, 4-cyclohexylcyclohexanol and positional isomers thereof, etc., examples of starting materials include phenol, o-, m- or p-cresol, xylenol, ethylphenol, o-t-butylphenol, p-t-butylphenol, 2,4-di(t-butyl)phenol, 2,6-di(t-butyl)phenol, p-phenylphenol, etc.

{Production of alicyclic diols}

Starting materials for producing 1,2-cyclohexanediol, 1,3-cyclohexanediol, 1,4-cyclohexanediol, 4,4'-bis(cyclohexanol), hydrogenated bisphenol A, and hydrogenated bisphenol Z include catechol, resorcin, hydroquinone, 4,4'-biphenol, bisphenol A, bisphenol Z, etc.

{Production of alicyclic diamine}

Starting materials for producing alicyclic diamines, such as 1,2-cyclohexanediamine, 1,3-cyclohexanediamine, 1,4-cyclohexanediamine 1,3-bis-aminomethyl-cyclohexane, 1,4-bis-aminomethyl-cyclohexane and 4,4'-methylene-bis(cyclohexylamine) include o-phenylenediamine, m-phenylenediamine, p-phenylenediamine, m-xylylenediamine, p-xylylenediamine, 4,4'-methylenedianiline, etc.

Disclosed is further the hydrogenation of heterocyclic ring of heterocyclic compounds having one or more (particularly 1 to 5) unsaturated bonds. Examples of such heterocyclic compounds include pyridine compounds such as pyridine, methyl nicotinate, quinoline compounds such as quinoline, and furan compounds such as furfuryl aldehyde, furan carboxylic acid, and the like. In particular, the process of the invention makes it possible to produce piperidine from pyridine, methyl hexahydronicotinate from methyl nicotinate, decahydroquinoline from quinoline, tetrahydrofurfuryl alcohol from furfuryl aldehyde, and tetrahydrofurfuryl carboxylic acid from furancarboxylic acid.

When using the above-mentioned aromatic compound or unsaturated heterocyclic compounds as starting materials, it is generally preferable to use a nickel-based solid catalyst or a noble-metal-based solid catalyst, which will be described later, for carrying out the heterogeneous catalytic hydrogenation process of the present invention.

<Production of amines from nitrile compounds and amide compounds>

Disclosed is further a process which is applicable to hydrogenation of nitrile compounds to amines. Nitrile compounds as a starting material are not limited, and include butyronitrile, lauronitrile, stearonitrile, etc.

Additionally, the process is applicable to hydrogenation of an amide compound to an amine. Amides as a starting material are not limited, and include butyramide, lauramide, stearamide, etc.

It is generally preferable to produce amines from nitrile compounds or amide compounds by carrying out the heterogeneous catalytic hydrogenation process of the present invention using a nickel-based solid catalyst or a noble-metal-based solid catalyst, which will be described later.

<Imide compound starting materials>

Disclosed is yet further the hydrogenation of imides to pyrrolidine compounds or amines. For example, $\Delta^4$-tetrahydrophthalimide and/or hexahydrophthalimide may be hydrogenated to produce octahydroisoindole.

It is generally preferable to produce a pyrrolidine compound and amine from an imide compound by the heterogeneous catalytic hydrogenation process of the present invention using a copper-based solid catalyst or a noble-metal-based solid catalyst, which will be described later.

<Sulfur and chlorine contents of a substance to be hydrogenated>

Examples of substances to be hydrogenated that may be subjected to the hydrogenation in the present invention include the above-described various compounds. In particular, used are such compounds in which the total content (by weight) of chlorine atom and sulfur atom is 5 ppm or less, preferably 3 ppm or less, particularly preferably 1 ppm or less, and still more preferably 0.3 ppm or less.

Compounds with a sum of chlorine atom and sulfur atom contents of 5 ppm or less do not require frequent replacement of solid catalyst, and renders the process more efficient.

According to the inventors' studies, the phenomenon of catalyst poisoning by chlorine atoms is completely different from that by sulfur atoms. More specifically, a sulfur compound reacts very quickly with a catalyst metal under hydrogenation conditions, and is localized in the uppermost part of the catalyst layer in the form of metal sulfide. In contrast, chlorine atoms may undergo migration. For example, a metal chloride produced by reaction with the catalyst metal has a tendency to dissolve in the reaction mixture, and then diffuses downward into the catalyst layer, whereby a poisoned zone (catalytically inactive zone) is expanded. Chlorinated organic compounds react slowly with the catalyst, and consequently reach deeper into the catalyst layer, resulting in the same behavior (migration) as above.

The inventors have found that starting materials whose total amount of chlorine and sulfur atoms is 5 ppm or less are advantageous in the presence of the two types of poisoning substances, sulfur and chlorine, whose behavior is completely different from each other.

{Causes of chlorine atom incorporation and methods for reducing chlorine atom content}

Causes of chlorine atom incorporation include cases in which inorganic compounds, such as salt (NaCl), chloride ion, etc. are incorporated, and cases in which chlorinated organic compounds are incorporated.

Salt mixing is a fundamental problem, particularly in the case where a substance to be hydrogenated is derived from a natural source such as plants, because many such natural sources need salt in the production of the substance to be hydrogenated. Chlorine atoms may also be incorporated when hydrochloric acid is used for neutralization of a transesterification or as an esterification catalyst. In general, inorganic compounds can be reduced and eliminated by washing with water.

On the other hand, in the step of separating or processing these raw materials (substances to be hydrogenated) from natural sources, chlorine compounds may react with such raw materials to form chlorinated organic substances. For example, beef tallow or lard are heated at high temperature with the blood and juice of dismembered cows and pigs in the manufacturing process, and salt is partly converted to organic chlorine, and thus fatty acids derived from beef tallow or lard contain organic chlorine. Recycled deep-frying vegetable-based oils (so-called used vegetable oil) also contain organic chlorine, as a result of contact with foods containing salt at high temperature. Fatty acids derived from coconut oil also contain organic chlorine of natural origin. These chlorinated organic compounds can be reduced or eliminated by precision distillation, because of their high molecular weight and low vapor pressure due to the atomic weight of chlorine, 35.4, which is about three-times that of carbon.

Although the use of starting materials which do not contain such chlorine is most desirable, the starting material can be selected in view of raw material cost, supply stability, etc.

{Causes of sulfur atom incorporation and methods for reducing sulfur atom content}

Sulfur atoms may be incorporated when p-toluenesulfonic acid or sulfuric acid is used for neutralization of a transesterification catalyst or as an esterification catalyst. In general, these compounds can be reduced and eliminated by washing with water.

In the case of starting materials originating from natural products, sulfur compounds originating from proteins, such as cystine, are contained. Beef tallow, lard, coconut oil, palm oil, and palm kernel oil contain such sulfur compounds originating from protein, and when they are used as such, sulfur compounds are incorporated into fatty acids and methyl esters, which are supplied as starting materials in the present invention. Sulfur compounds originating from protein can be reduced or eliminated by distillation.

In the case of starting materials derived from petroleum or coal, sulfur compound content usually depends on the manufacturing process of the upstream raw materials of such starting materials, and therefore it is preferable to select raw materials. For example, $\Delta^4$-tetrahydrophthalic anhydride or $\Delta^4$-3-methyl tetrahydrophthalic anhydride to be used in carbon-carbon double bond hydrogenation are manufactured by Diels-Alder reaction of a conjugated diene compound with maleic anhydride. Such conjugated diene compounds, such as butadiene or isoprene, often contain sulfur compounds, such as carbon disulfide, and thus appropriate starting material selection is preferred. For nuclear hydrogenation of aromatic compounds, it is preferable to select a petroleum-derived starting material purified by deep desulfurization and having extremely low sulfur content. Starting materials derived from coal often contain a large quantity of sulfur compounds such as thiophene, and therefore it is preferable to avoid using them.

Solid catalyst

Known solid catalysts used for hydrogenation can be used as a solid catalyst for use in the present invention. In the present invention the solid catalysts is a zinc-based catalyst. In such solid catalysts, at least one metal selected from the group consisting of chromium, molybdenum, tungsten, magnesium, barium, aluminum, calcium, zirconium, manganese, nickel, silicon, and oxides thereof may additionally be supported as a promoter.

Examples of supports used for such solid catalysts include silica, alumina, silica-alumina, titania, diatomaceous earth, kaolin, activated carbon, carbon, graphite, zeolite, montmorillonite and like clays; alkaline earth metal silicates, etc.

The solid catalysts used in the present invention are known per se. These catalysts may be used as such, but are preferably subjected to a suitable activation treatment, such as reduction treatment, prior to use. Such activation treatment as reduction treatment can be carried out by a conventional method.

<Copper-based catalyst>

Examples of copper-based catalysts include at least one catalysts selected from copper, copper-zinc, copper-chromium, copper-zinc-chromium, and oxide thereof, and solid catalysts in which a modified catalyst is supported, the modified catalyst being prepared by adding molybdenum, tungsten, magnesium, barium, aluminum, calcium, zirconium, silicon and oxides thereof to the copper-based catalyst.

More specifically, there may be mentioned solid catalysts comprising copper oxide, copper-zinc-chromium oxide, copper-chromium-zinc-magnesium oxide, copper-zinc-chrominum-barium oxide, copper-zinc oxide, copper-zinc-magnesium oxide, copper-zinc-aluminum oxide, copper-chromium oxide, copper-chromium-magnesium oxide, copper-chromium-manganese oxide, copper-chromium-barium oxide, copper-chromium-barium-magnesium oxide, copper-chromium-manganese-barium oxide, copper-chrominum-manganese-magnesium oxide, etc.

Moreover, there may also be mentioned alkaline earth metal silicate-supported solid catalyst, such as copper-calcium-silicic acid, copper-manganese-silicic acid, copper-barium-silicic acid, copper-calcium-barium-silicic acid, or copper-calcium barium-manganese-silicic acid.

These copper-based solid catalysts are suitable for the reduction reaction of saturated or unsaturated as disclosed above fatty acid esters to saturated alcohols.

In this specification and Claims, the expression "M1-M2 oxide" such as the above-mentioned copper-chromium oxide denotes a catalyst comprising an oxide of metal M1 and oxide of metal M2. For example, "copper-chromium oxide" denotes a catalyst comprising a copper oxide and a chromium oxide. Similarly, "M1-M2-M3 oxide" such as copper-zinc-aluminum oxide denotes a catalyst comprising an oxide of metal M1, an oxide of metal M2, and an oxide of metal M3. For example, "copper-zinc-aluminum oxide" denotes a catalyst comprising a copper oxide, a zinc oxide, and an aluminum oxide. The same applies to other similar expressions.

<Zinc-based catalyst>

Examples of zinc-based catalysts include zinc-chromium oxide, zinc-aluminum oxide, zinc-aluminum-chromium oxide, zinc-chromium-manganese oxide, zinc-iron oxide, zinc-iron-aluminum oxide, etc. In the invention, these zinc-based solid catalysts are used for the reduction of unsaturated fatty acid esters to unsaturated alcohols.

<Nickel-based catalyst>

Examples of nickel-based catalysts are nickel-diatomaceous earth, nickel-chromium oxide, etc. These nickel-based solid catalysts are suitable for double bond hydrogenation and aromatic nucleus hydrogenation.

<Noble-metal-based catalyst>

Noble-metal-based solid catalysts containing, for example, ruthenium, palladium, platinum, rhodium or oxides thereof include solid catalysts in which such metal or oxide thereof is supported by a support, such as silica, alumina, silica-alumina, titania, activated carbon, carbon, graphite or the like. These noble-metal-based solid catalysts are effective in various hydrogenations, and in particular are suitable for double bond hydrogenation and aromatic nucleus hydrogenation.

<Catalyst shape>

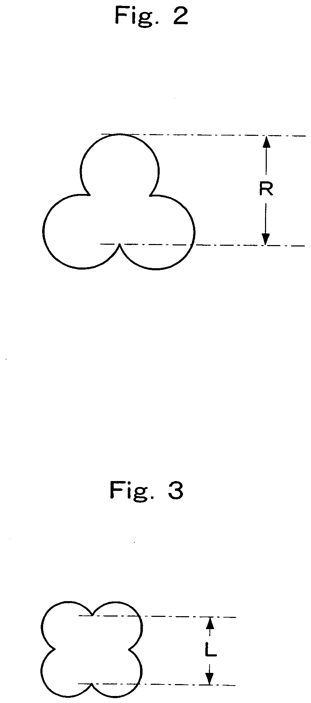

The shape of the solid catalysts used in the present invention is preferably a cylinder, a hollow cylinder, a trilobal-shaped rod, a tetralobal-shaped rod, sphere, etc.; and two or more solid catalysts of different shapes may be used.
In particular, solid catalysts with a minimum length of about 1 to about 10 mm are recommended. In the specification and claims, "minimum length" is determined as follows: For example, in the case of a spherical-shaped catalyst with a diameter of 5 mm, the minimum length is the diameter (5 mm), and in the case of a cylindrical-shaped catalyst with a diameter of 3 mm and height of 5 mm, the minimum length is the diameter (3 mm). In the case of a hollow cylinder, e.g., with an outer diameter of 3 mm (inner diameter of 2 mm) and height of 5 mm, the minimum length is not inner diameter but the outer diameter (3mm). In the case of a trilobal-shaped rod, the size (R) shown in Fig. 2 is the minimum length, and in the case of a tetralobal-shaped rod, the size (L) shown in Fig. 3 is the minimum length (in each case, the height (the length in the axial direction) is greater than R or L).
When the minimum length exceeds 10 mm, the catalytic exterior surface area of the solid catalyst in a reactor is reduced and thus productivity tends to be decreased. In contrast, when the minimum length is less than 1 mm, solid catalysts become more closely packed, increasing a pressure loss and making it hard to form trickle flow.

<Production process of a catalyst>

The production processes of these solid catalysts are not limited, and include conventional processes, such as tumbling granulation, compression molding, extrusion molding, melt granulation, etc., and, a solid catalyst can be easily obtained with a tablet press, a granulator, an extrusion molding machine, oil-spherical molding apparatus from a powder or paste.

<Catalyst strength>

It is essential in the present invention to charge and use a solid catalyst with a catalyst strength of 1.0 kg to 4.0 kg per catalyst.
The catalyst strength in the present invention is a strength determined by measuring the minimum crushing strength each of 100 catalyst samples and calculating the mean value A and the standard deviation value ($\sigma$) and calculating said strength using the formula: $A-2\sigma$. In the present invention, it is important that the catalyst strength is 1.0 kg to 4.0 kg, and in particular 1.5 to 4.0 kg.
In this specification, the minimum crushing strength is measured according to "3.1 Test method for crushing strength" of JIS Z-8841-1993.
In the present invention, the "minimum crushing strength" refers to the smaller of two crushing strengths, one being a crushing strength determined by compressing, in the vertical direction (the axial direction), the solid catalyst used in the present invention in the form of a cylinder, hollow cylinder, trilobal-shaped rod, or tetralobal-shaped rod, and the other being a crushing strength determined by compressing such solid catalyst in the transverse direction (the radial direction, i.e., direction perpendicular to the axial direction). In general, the crushing strength is weakest in the transverse direction (the direction perpendicular to the axial direction), when the solid catalyst is in the form of a cylinder, hollow cylinder, trilobal-shaped rod, or tetralobal-shaped rod.
With respect to solid catalysts having a highly symmetric shape, such as a sphere or a cube, there is generally no difference in crushing strength with different compression direction, and thus, in this specification, the crushing strength measured according to the process of the above-mentioned JIS standard is the minimum crushing strength.
When the above-mentioned catalyst strength is lower than 1.0 kg, the catalysts tend to be damaged to cause catalyst outflow, and in a worse case, cause clogging of a reactor and connecting piping. Even with respect to catalysts having a high mean value (A), if the standard deviation value ($\sigma$) is high (i.e., high proportion of weak catalysts), these weak catalysts may be damaged, resulting in catalyst outflow and a system clogging. Therefore, effective solid catalysts are those designed considering strength variation as well. Such problems do not arise in the present invention.
The catalyst strength obtained by the formula $A-2\sigma$ represents statistically that 97.5% or more of the solid catalysts out of 100 catalyst samples had a minimum crushing strength of 1 kg or more.

Hydrogenation process

The heterogeneous catalytic hydrogenation process of the present invention is carried out by flowing hydrogen gas and a substance to be hydrogenated co-currently downward from above in the presence of the above-mentioned catalyst using the substance to be hydrogenated as a starting material, under trickle flow condition. The details will be described below.

The trickle flow of the present invention denotes the following state. A liquid phase (i.e., a substance to be hydrogenated in a liquid form) flows down in a film over the solid catalysts by the action of gravity, and hydrogen gas flows in the form of a continuous flow in parallel with the liquid flow through a space between the liquid phase flowing down in a film over the surface of a catalyst grain and the liquid phase flowing down in a film over the surface of a horizontally adjacent solid catalyst grain.

One example is shown in Fig. 1. In Fig. 1, liquid phase 100 flows in a film over the surface of solid catalyst grain 102, flows down in a film over the surface of solid catalyst grain 103 located below the solid catalyst grain 102, and then flows down in a film over the surface of next lower solid catalyst grain 104 (and of the subsequent solid catalyst grain). In the same manner, the liquid phase 100 flows in a film over the surface of a solid catalyst grain 110 adjacent to the solid catalyst grain 102, and flows down in a film over the surface of next solid catalyst grain 111 therebelow (and of the subsequent solid catalyst grains).

Hydrogen gas flows down in the form of a continuous flow in the space S between the film-like liquid phase flowing down over the surfaces of the solid catalyst grains 102, 103, and 104 (and subsequent solid catalyst grains), and the film-like liquid phase flowing down over the surfaces of the solid catalyst grains 110 and 111 (and subsequent solid catalyst grains) adjacent to these solid catalyst grains 102, 103, and 104 (and subsequent solid catalyst grains). In Fig. 1, spherical solid catalysts are illustrated, but the above description similarly applies to solid catalysts having other shapes.

The trickle flow condition as described above varies depending on a tube diameter, starting-material feed rate, hydrogen gas feed rate, a hydrogen gas pressure, a catalyst size, etc.

When the solid catalyst of the present invention is used, the trickle flow is attained under the following condition: starting-material feed rate is in the range of about 0.4 to about 40 $m^3$/h (preferably about 1 to about 30 $m^3$/h, and more preferably about 2 to about 30 $m^3$/h) per square meter of the cross-sectional area of a reaction tower under the reaction condition, and hydrogen gas feed rate is in the range of about 4 to about 4,000 $m^3$/h (preferably about 10 to about 2,000 $m^3$/h, and more preferably about 40 to about 1,000 $m^3$/h) per square meter of the cross-sectional area of a reaction tower under the reaction condition.

When the hydrogen gas feed rate is greater than the above-described range, the liquid phase (the substance to be hydrogenated) partially flows in the form of drops (i.e., a spray flow), and thus high yield is not attained as expected by the invention. When the starting material feed rate is greater than the above-described range, the liquid phase turns into pulsing flow. The pulsing flow is unfavorable for the heterogeneous catalytic hydrogenation process of the present invention, since the solid catalyst grains collide with each other and disintegrate due to the increased pressure loss and fluidization of the solid catalyst. At a further increased starting material feed rate, the starting material turns into a continuous phase in which gas disperses to form bubbles (bubble flow), failing to attain high productivity as expected by the present invention.

In addition to the above-described trickle flow condition, it is important in the present invention that the dynamic liquid phase holdup amount per unit catalyst exterior surface area under the reaction conditions is about $0.005 \times 10^{-3}$ to about $0.14 \times 10^{-3}$ $m^3/m^2$, and preferably about $0.05 \times 10^{-3}$ to about $0.12 \times 10^{-3}$ $m^3/m^2$, after hydrogenation reaches steady state. When the dynamic liquid phase holdup amount per catalyst exterior surface area is greater than $0.14 \times 10^{-3}$ $m^3/m^2$, pulsing flow tends to occur, in which portions with excessively large liquid phase holdup amount and portions with smaller liquid phase holdup amount flow in turn. In contrast, if the dynamic liquid phase holdup amount per unit catalyst exterior surface area is smaller than $0.005 \times 10^{-3}$ $m^3/m^2$, high productivity is not pracitically obtained due to excessively slow feed rate, or the above-mentioned spray flow arises due to excessively large gas flow.

The dynamic liquid phase holdup amount per unit catalyst exterior surface area can be adjusted to the range from about $0.005 \times 10^{-3}$ to about $0.14 \times 10^{-3}$ $m^3/m^2$ by adjusting the size (minimum length), shape, etc., of the solid catalyst to be charged, in addition to adopting the above-mentioned trickle flow condition.

The "catalyst exterior surface area" according to the invention denotes the exterior surface area of the solid catalyst, and is the macroscopic surface area of the solid catalyst grains. More specifically, when a catalyst is in the shape of a cylinder with radius, "r" and height, "h", the catalyst exterior surface area is the sum of the upper and lower circular areas, $2\pi r^2$, and the lateral surface area, $2\pi rh$. In a spherical-shaped catalyst with radius, "r", the exterior surface area is $4\pi r^2$. The above-mentioned term is used in the specification so as to be distinguished from the microscopic surface area including catalyst pores. Accordingly, "per unit catalyst exterior surface area" denotes "per square meter of the total area of the catalyst exterior surface areas of the catalysts charged in a reaction tower".

The "dynamic liquid phase holdup amount" in the present invention denotes the amount obtained by subtracting the static holdup amount from the total holdup amount.

In a practical reaction tower, the reaction liquid in the vicinity of the points where solid catalysts contact with each other is in a substantially stationary state because such reaction liquid is shielded by the solid catalysts from hydrogen gas flowing down co-currently therewith and also due to surface tension or other factors, and contribute less to production. Accordingly, this reaction liquid is not taken into consideration. The amount thereof will be referred to as "static holdup amount".

Referring to Fig. 1, for example, in the space between the solid catalyst 102 and the solid catalyst 103, the liquid phase (substance to be hydrogenated) 100, shielded by the solid catalyst 102 and under the action of surface tension or other factors, is substantially immobilized in the space between the solid catalyst 102 and the solid catalyst 103. The amount of such liquid phase trapped and substantially immobilized is referred to as "static liquid phase holdup amount".

The dynamic liquid phase holdup amount per catalyst exterior surface area of the present invention denotes a total amount of the liquid phase that flows down in a film over the surfaces of each catalyst in co-current with hydrogen gas also flowing down. Accordingly, the sum of the static liquid phase holdup amount and the dynamic liquid phase holdup amount is the total holdup amount.

In a real process, the dynamic liquid phase holdup amount can be determined by measuring the liquid weight (W) discharged from the bottom of the reaction tower when suspending supply of the liquid phase while maintaining supply of the hydrogen gas under the reaction conditions. That is to say, W is the dynamic liquid phase holdup amount.

In the present invention, it is preferable that the dynamic liquid phase holdup amount per catalyst exterior surface area is $0.005 \times 10^{-3}$ to $0.14 \times 10^{-3}$ m$^3$/m$^2$, as mentioned above, and the dissolved hydrogen concentration in the liquid phase is 0.01 to 5.0 kmol/ m$^3$, and in particular 0.3 to 5.0 kmol/m$^3$ It is also preferable in the present invention that the dissolved hydrogen concentration in the liquid phase is maintained within 10 to 100% of the saturated hydrogen concentration at the point 1 m below the highest point of the catalyst layer in the reaction tower after hydrogenation reaches steady state.

It is preferable that the dissolved hydrogen concentration in the liquid phase is maintained within 10 to 60% of the saturated hydrogen concentration in the liquid phase, at the point 1 m below the highest point of a catalyst layer in the reaction tower, in processes involving vigorous reactions, e.g., processes for hydrogenating many double bonds, such as a process for hydrogenating unsaturated fatty acid alkyl esters

Moreover, it is preferable to maintain the dissolved hydrogen concentration in the liquid phase within 50 to 100% of the saturated hydrogen concentration in the liquid phase, at the point 1 m below the highest point of the catalyst layer in the reaction tower, in processes involving very small reaction change, for example: processes in which a catalyst with low activity is selected so as to retain reaction selectivity, such as a process for producing unsaturated alcohols from unsaturated carboxylic acid esters

Herein, the above-mentioned dissolved hydrogen concentration and saturated hydrogen concentration in the liquid phase each denote the concentrations at a temperature and a pressure of the hydrogenation reaction in question.

In the present invention, "saturated hydrogen concentration in the liquid phase" denotes the maximum hydrogen concentration, i.e., the greatest possible amount of hydrogen dissolvable in the reaction substrate itself, and is determined by bringing hydrogen gas into full contact with the liquid phase in the absence of catalysts under the reaction conditions to be employed.

The dissolved hydrogen concentration according to the present invention is the concentration of hydrogen in the liquid phase contacting the solid catalyst together with the substance to be hydrogenated as a result of dissolution of gas phase hydrogen molecules in the liquid phase. More specifically, the dissolved hydrogen concentration, is controlled by various dynamic factors, as a result of a combination of various complicated factors, such as rate of dissolution of hydrogen gas into the liquid, rate of diffusion of hydrogen gas in the liquid phase to solid catalyst surface, rate of bringing hydrogen gas to its active state on the solid catalyst surface, rate of reaction of hydrogen in the active state with the reaction substrate, etc., and then hydrogen is detected as hydrogen molecules that have been "dissolved" in the liquid phase.

In the invention, the dissolved hydrogen concentration is preferably maintained within 10 to 100% of the saturated hydrogen concentration. Preferably, the dissolved hydrogen concentration is always 100% of saturated hydrogen concentration, and in reality, the concentration is 100% in the lower part of the reaction tower. In contrast, in the upper part of the reaction tower, the amount of reaction of a substrate and hydrogen molecules tends to be very large, and dissolved hydrogen concentration tends to be lower. According to the inventors' research, it has been found that the dissolved hydrogen concentration in the liquid phase at the point 1 m below the top of the reaction tower, when maintained at 10% or higher, is advantageous for achieving high productivity.

The dissolved hydrogen concentration is adjusted to 0.01 to 5.0 kmol/m$^3$, and the dissolved hydrogen concentration in the liquid phase at the point 1 m below the highest point of the catalyst layer in the reaction tower is maintained within 10 to 100% of the saturated hydrogen concentration in the liquid phase as follows.

The range of dissolved hydrogen concentration, and the ratio of dissolved hydrogen concentration to saturated hydrogen concentration are influenced by an extremely wide range of factors, and thus various methods can be adopted. Such methods can be easily carried out considering the following factors. Specifically, in respect of catalyst, selection of a low-activity catalyst, reduction in the concentration of catalytically active species, reduction in catalyst exterior surface area, or reduction in dynamic liquid phase holdup amount tends to increase hydrogen concentration. In respect of hydrogen gas feed method, increase in hydrogen pressure or increase in hydrogen flow rate tends to increase dissolved hydrogen concentration. In respect of the method of supplying substances to be hydrogenated, reduction in reaction substrate concentration or reduction in feed amount of reaction starting material tends to increase dissolved hydrogen concentration. Accordingly, the foregoing adjustments can be easily carried out by determining the relationship between these factors and the above-mentioned dissolved hydrogen concentration (and additionally the ratio of the dissolved hydrogen concentration to the saturated hydrogen concentration) under the reaction conditions to be employed, and suitably selecting these factors according to product type.

<Reaction temperature, reaction pressure, reaction solvent, etc.>

Reaction temperature and reaction pressure for hydrogenation are not limited insofar as the hydrogenation can be completed, and a practical reaction rate is obtained when the reaction temperature is in the range, in general, of about 50 to about 350°C, preferably about 50 to about 300°C. The reaction pressure is generally in the range of atmospheric pressure to about 35 MPa, preferably in the range of about 0.9 to about 30 MPa.

The above-mentioned hydrogenation is usually carried out in the absence of solvents. However, a solvent may be used when the starting material and/or reaction product has a high melting point and are/is difficult to handle, or for the purpose of improving reactivity or selectivity, or for efficient elimination of reaction heat, etc.

When the melting point of the starting material is high and the melting point of the reaction product is low, the use of the reaction product itself as a solvent is effective. In the case of a nuclear hydrogenation in which the heat of reaction is very high, reaction starting material may be fed as diluted with a solvent or the reaction product itself.

The solvent is suitably selected from those which are inert to hydrogenation and do not react with the starting material and reaction product. More specifically, water, alcohols having 1 to 10 carbon atoms, diols, such as ethylene glycol, propylene glycol, etc., ether solvents, such as diglyme, triglyme, etc., ether alcohols, such as methyl propylene glycol, butyl cellosolve, etc., paraffins having 5 to 10 carbon atoms, and cycloparaffin hydrocarbons, etc., can be used. Aromatic hydrocarbons, such as toluene, xylene, etc., can also be used in reactions other than nuclear hydrogenation.

The amount of solvent used is not limited and is generally in the range of 0.05 to 100 weight parts, preferably in the range of 0.1 to 50 weight parts, per weight part of starting material substance to be hydrogenated on a weight basis.

< Reaction tower >

In the invention, a reaction tower of any desired form can be used insofar as uniform trickle flow can be formed when solid catalyst is charged. Such uniform trickle flow can be formed by providing, at the upper part thereof, a bubble cap type distribution plate or other equipment which uniformly supplies liquid to the solid catalyst. For the purpose of preventing channeling, it is preferable to connect, in series, relatively short reaction towers equipped with a distribution plate, or to adopt a long reaction tower equipped with re-distribution device or devices.

The length and diameter of the reaction apparatus are suitably selected according to the kind of starting material, reaction type, production amount, construction cost of the apparatus, operation efficiency, etc. In general, it is preferable to use a reactor with a diameter of about 2 to about 200 cm, particularly about 3 to about 100 cm, and a catalyst layer length of about 2 to about 20 m, particularly about 3 to about 15 m.

A multi-tube reactor can also be used as a reaction apparatus, and in that case it is preferable to use a multi-tube reactor comprising 10 to 1000, preferably 20 to 500, tubular reactors, each having a diameter of about 2 to about 20 cm, preferably about 2 to about 10 cm and having a catalyst layer length of about 2 to about 20 m, preferably about 3 to about 15 m.

For the purpose of reaction heat elimination, it is preferable to carry out the process after connecting a piping for introducing hydrogen gas for cooling to midpoint of the reaction tower, or after connecting a heat exchanger for eliminating heat to be evolved during the most intense reaction period in series to the reaction tower.

EXAMPLES

The present invention will be described below in detail with reference to Examples and Comparative Examples, but is not limited to these examples.

In each Example and Comparative Example, "%" means "weight%", unless otherwise specified.

1) Apparatus

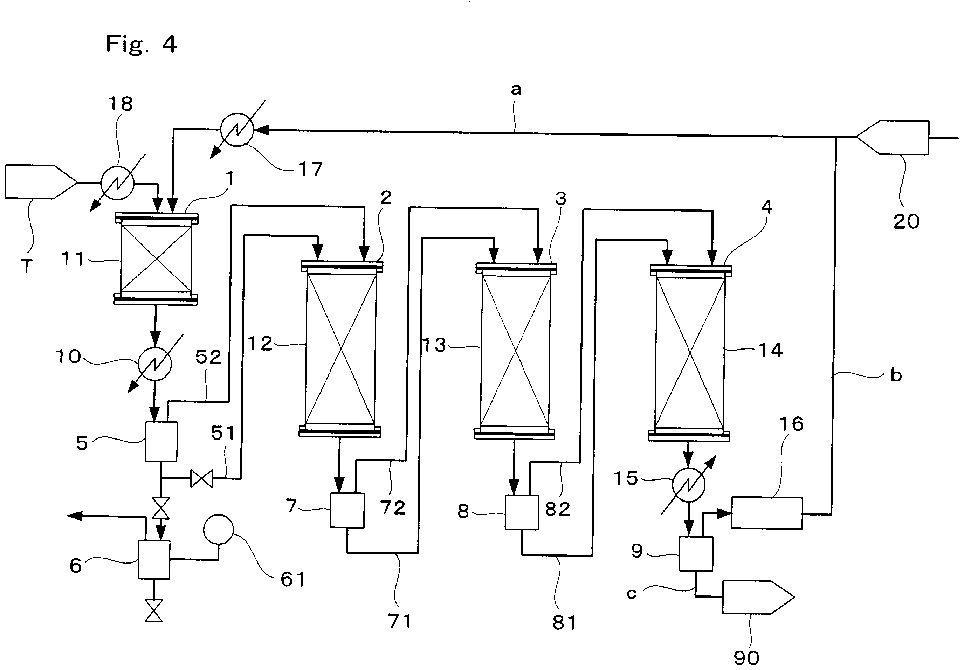

An apparatus as shown in Fig. 4 was employed. More specifically, a reactor 1 with a diameter of 40 cm and with a catalyst charge height of 1 m, and reactors 2, 3 and 4 each with a diameter of 40 cm and with a catalyst charge height of 3 m were connected in series via high-pressure gas-liquid separators 5, 7, and 8. Each reaction tower is provided with a horizontal bubble cap-type distribution plate (not shown) in its upper part.
The following operation was performed in Examples and Comparative Examples below. Hydrogen gas pressurized to a predetermined pressure was made to flow using a hydrogen gas compressor 20 and a hydrogen gas circulator 16 at the predetermined flow rate through the reaction towers 1, 2, 3, and 4, which had previously been charged with catalysts activated in the hydrogen current. A temperature elevation was initiated, and at a temperature 20 to 50°C below the predetermined reaction temperature, feeding of a starting material was commenced, and temperature elevation was continued. Hydrogenation products were continuously withdrawn from a piping c for removing hydrogenation products that was connected to a high-pressure gas-liquid separator 9, and were then accommodated in a crude reaction product tank 90. Reaction onset time is the time for the temperature to reach the predetermined temperature. Hydrogen gas was newly supplied continuously in an amount equivalent to the amount of hydrogen consumed by the hydrogenation, so as to maintain the predetermined pressure. Usually, a steady state was reached in about 10 hours.
In the following Examples, the reactor 1 with a catalyst charge height of 1 m was used, and thus the dissolved hydrogen concentration at the point 1 m below the highest point of the catalyst layer in the reaction tower could be measured. However, an integrated reaction tower provided with a re-distribution device may be used when the process is industrially carried out.

2) Measurement of conversion

Conversion was determined using the crude reaction end-product withdrawn from the high-pressure gas-liquid separator 9 connected to the end part (namely, the reaction tower 4) of a reaction tower composed of the reactors 1, 2, 3, and 4 (the sum of the heights of the catalyst charged layers 11 to 14: 10 m).
In producing saturated or unsaturated alcohols by ester reduction, conversion was determined based on the measurement result of a saponification value (JIS K-0070) or an iodine value (JIS K-0070). In nuclear hydrogenation reaction, conversion was obtained based on GLC measurement result.

3) Measurement of dynamic liquid phase holdup amount per unit catalyst exterior surface area

A low-pressure gas-liquid separator 6 was connected to the bottom of the high-pressure gas-liquid separator 5 connected to the reactor 1 with a catalyst charge height of 1 m. The dynamic liquid phase holdup amount per unit catalyst exterior surface area under trickle flow condition was measured by temporarily suspending transfer from the high-pressure gas-liquid separator 5 to the next reactor 2, and capturing the entire amount of the liquid retained by the catalysts in the reactor with the low-pressure gas-liquid separator 6.
Catalyst exterior diameter surface area was calculated based on the mean value of measured dimensions (diameter and height) for ten catalysts to be used.

4) Measurement of the dissolved hydrogen concentration in the liquid phase at the point 1 m below the highest point of the catalyst layer at the top part of a reaction tower

The low-pressure gas-liquid separator 6 was connected to the bottom of the high-pressure gas-liquid separator 5 connected to the reactor 1 with a catalyst charge height of 1 m, and reaction liquid (liquid phase) was hermetically withdrawn. The number of moles of dissolved hydrogen was calculated by measuring the amount of hydrogen gas dissolved under high-pressure based on the pressure change shown by an installed pressure gauge.
More specifically, the dissolved hydrogen concentration in the reaction liquid (liquid phase) in the high-pressure gas-liquid separator 5 corresponds to the concentration governed by the reaction condition in the reactor 1 (i.e., at the point 1 m below the highest point of the catalyst layer). When the liquid phase in the reactor 1 is transferred to the low-pressure gas-liquid separator 6, hydrogen molecules dissolved in the liquid phase are liberated in gaseous state, and detected by the gauge needle change (pressure increase) of a pressure gauge 61 installed. The dissolved hydrogen concentration in the liquid phase in the high-pressure gas-liquid separator 5 was calculated based on space volume of the low-pressure gas-liquid separator 6 and the pressure increase.
Hereinafter, "the point 1 m below the highest point of a catalyst layer at an upper part of the reaction tower is abbreviated as "the 1 m point of the upper part of the reaction tower".

5) Measurement of saturated hydrogen concentration in the liquid phase

The reaction liquid containing no catalyst withdrawn by the method described in item 4) above was placed in a batch autoclave equipped with a stirrer, and after supplying hydrogen gas with stirring, the saturated hydrogen concentration in the liquid phase at the temperature and pressure in question was measured.

6) Measurement of catalyst strength

Using a Kiya digital hardness tester (manufactured by KIYA SEISAKUSHO LTD., trade name "KHT-20") and according to the method described in "3.1 Test method for crushing strength" of JIS Z-8841-1993", crushing strengths of a cylindrical solid catalyst employed in each Example was measured by pressing the same in the vertical direction (the axial direction) and in the transverse direction (the direction perpendicular to the axial direction), and the two different crushing strengths measured were compared.

The measurement principle of the above-mentioned crushing strength was as follows: one solid catalyst grain as a measurement sample was placed on a stationary sample-holder with a diameter of 25 mm, and a movable pressing tip with a diameter of 5 mm was lowered at a speed of 1 mm/second to press the measurement sample, and the crushing strength was measured when the solid catalyst was destroyed. The specification of the measurement apparatus was as follows: crushing strength detection mechanism = capacitance sensor single point load cell, and reading limit = 0.05 kgf.

With respect to the solid catalysts employed in all Examples, the crushing strength measured by pressing in the transverse direction was smaller than that measured by pressing in the vertical direction, and thus the crushing strength measured in the transverse direction was taken to be the "minimum crushing strength".

The solid catalyst minimum crushing strength was measured with respect to 100 solid catalyst samples, and the mean value A and standard deviation $\sigma$ were calculated. The value calculated by the following formula was defined as the catalyst strength (kg):

$$\texttt{Catalyst strength (kg) = A-2}\sigma$$

In the formula, "A" denotes the mean of the minimum crushing strength values and "$\sigma$" denotes the standard deviation of the minimum crushing strength values.

In the following Examples and Comparative Examples, the values of starting-material feed rate, hydrogen gas feed rate, and dynamic liquid phase holdup amount are values under the reaction conditions.

Example 1 Hydrogenation of a saturated fatty acid ester to a saturated alcohol (Reference)

The reactors 1, 2, 3, and 4 were charged with a cylindrical copper-chromium oxide catalyst (bulk specific gravity of 1.1kg/l, diameter of 3 mm, height of 3 mm) manufactured with a tabletting machine.

For activating the catalyst, a hydrogenation treatment was carried out in advance. More specifically, the air in the reactor was replaced with nitrogen gas, and the temperature was raised to 150°C under a nitrogen stream. A Nitrogen-hydrogen gas mixture was made to flow while increasing hydrogen gas concentration gradually so that the hydrogen gas concentration became 100% in 10 hours. After the hydrogen gas concentration reached 100%, the temperature was raised to 220°C. The hydrogen gas circulator 16 was actuated, and the hydrogen gas feed rate was made to be equivalent to the predetermined rate to be described below, immediately before commencing starting material feed.

Methyl laurate (sulfur content of 0.05 ppm, chlorine content of 0.20 ppm) derived from palm kernel oil was placed in a starting material tank T. Starting materials and hydrogen gas were fed to the reactors 1, 2, 3, and 4 via the high-pressure gas-liquid separators 5, 7, and 8 in succession at a starting material feed rate of 15 m$^3$/h per square meter of cross sectional area of the reaction tower and a hydrogen gas rate of 800 m$^3$/h per square meter of cross sectional area of the reaction tower, and at a temperature of 220°C and a pressure of 18MPa, and the operation was carried out under trickle condition for 500 hours, giving lauryl alcohol (crude reaction product) as a hydrogenation product.

Reaction results are shown in Table 1.

Table 1

| Operation time | Saponification value conversion (mol%) |
|---|---|
| after 100 hours | 99.0 |
| after 300 hours | 99.0 |
| after 500 hours | 99.0 |

The saponification value conversion was measured based on the saponification value of the crude reaction product ($SV_x$) and the saponification value of the starting material ($SV_0$) according to the following formula (1) (the same applies to the subsequent tables).

$$\texttt{Saponification value conversion (mol\%)}$$

$$\texttt{= [(SV}_0\texttt{-SV}_x\texttt{) /SV}_0\texttt{]} \times \texttt{100} \qquad\qquad \texttt{(1)}$$

It was confirmed that a steady state had been reached 10 hours after the commencement of the reaction. At that time, a crude reaction product liquid phase as withdrawn in a predetermined amount and transferred to the low-pressure gas-liquid separator 6 connected to the high-pressure gas-liquid separator 5 disposed at the 1 m point of the upper part of the reaction tower, and the dynamic liquid phase holdup amount and dissolved hydrogen concentration were then measured.

The dynamic liquid phase holdup amount per unit catalyst exterior surface area was $0.09 \times 10^{-3}$ $m^3/m^2$. The dissolved hydrogen concentration was 0.8 $kmol/m^3$.

Subsequently, 500 ml of the crude reaction product liquid phase thus obtained was placed in a 1,000 ml batch autoclave, and hydrogen gas was supplied with stirring, and the temperature was raised to 220°C, and the saturated hydrogen concentration at 18 MPa was then measured, and was found to be 1.5 $kmol/m^3$. That is to say, the dissolved hydrogen concentration at the 1 m point of the upper part of the reaction tower was 53% of the saturated hydrogen concentration in the liquid phase.

The strength of the charged catalyst was weakest in the transverse direction, and the mean value A of the minimum crushing strength was 6.1 kg and $\sigma$ was 2.0, so that (A-2$\sigma$) was 2.1kg. The solid catalyst had not disintegrated after 500 hours' operation, nor was any change involving pressure loss observed.

Comparative Example 1

The same operation was conducted as in Example 1 except that at a hydrogen gas rate of 5,000 $m^3/h$, the starting material feed rate per square meter of the cross section area of the reactor was set to 50 $m^3/h$, which is a flow rate causing pulsing flow.

The dynamic liquid phase holdup amount per unit catalyst exterior surface area was $0.15 \times 10^{-3}$ $m^3/m^2$. After 50 hours, the pressure in the high-pressure gas-liquid separator 9 connected to the reactor 4 dropped, whereas the pressure of the piping for supplying hydrogen gas to the reactor 1 was rising. The reaction was discontinued, and the reactor was opened and the catalyst was inspected. It was found that the catalyst grains at the bottom of each reactor had been disintegrated and the piping at the bottom part was almost clogged.

Comparative Example 2

Reactors 1 to 4 were charged with a cylindrical copper-chromium oxide catalyst (diameter of 3 mm and height of 3 mm). The strength of the charged catalyst was weakest in the transverse direction, and the mean value A of the minimum crushing strength was 5.2 kg and $\sigma$ was 2.5, and thus (A-2$\sigma$) was 0.2 kg.

The same operation was conducted as in Example 1 except that such a catalyst was used. After 200 hours, the pressure of the high-pressure gas-liquid separator 9 connected to the reactor 4 dropped, whereas the pressure of a piping for supplying hydrogen gas to the reactor 1 was increasing. By discontinuing the reaction, opening the reactor and inspecting the catalyst, it was found that the catalyst at the bottom of each reactor had been disintegrated and the piping at the bottom part was almost clogged.

Comparative Example 3

Reactors 1 to 4 were charged with a cylindrical copper-chromium oxide catalyst (diameter of 3 mm and height of 3 mm). The strength of the charged catalyst was weakest in the transverse direction, and the mean value A of the minimum crushing strength was 8.1 kg and $\sigma$ was 4.0, and thus (A-2$\sigma$) was 0.1 kg.

The same operation was conducted as in Example 1 except that such a catalyst was used. After 100 hours, the pressure of the high-pressure gas-liquid separator 9 connected to the reactor 4 dropped, whereas the pressure in the piping for supplying hydrogen gas to the reactor 1 was increasing. By discontinuing the reaction, opening the reactor and inspecting the catalyst, it was found that the catalyst at the bottom of each reactor had been disintegrated and the piping at the bottom part was almost clogged.

Example 2: Hydrogenation of an unsaturated fatty acid alkyl ester to a saturated alcohol (Inventive)

Fatty acid obtained by hydrolysis of palm oil was distilled, and then subjected to a fractionated solidification by cooling, giving unsaturated fatty acid (iodine value 98.3, glc composition C14: 0.6%, C16: 5.0%, C18F0: 1.8%, C18F1: 74.5%, C18F2: 18.0%, C20F1: 0.1%; in this specification, the expression (Fn), such as F1, F2, or the like shows that the unsaturated fatty acid has n double bonds. The same applies hereinafter.) The obtained unsaturated fatty acid was esterified with methyl alcohol using p-toluenesulfonic acid, and the methyl ester product after having been washed with water was used as a starting material (sulfur content of 0.5 ppm, chlorine content of 0.7 ppm).

Operation was conducted continuously under trickle flow condition for 500 hours in the same manner as in Example 1 except that the hydrogen gas rate per square meter of the cross sectional area of the reaction tower was set to 400 $m^3$/h, whereby crude palm alcohol was obtained as a hydrogenation product. The reaction advanced without any problem. The reaction results are shown in Table 2.

Table 2

| Operation time | Saponification value conversion (mol%) | Iodine value of reaction product (100 g$I_2$/g) |
|---|---|---|
| after 100 hours | 99.0 | 0.1 |
| after 300 hours | 99.0 | 0.1 |
| after 500 hours | 99.0 | 0.1 |

It was found that a steady state had been attained 10 hours after the start of the reaction. At that time, a crude reaction product liquid phase was withdrawn in a predetermined amount and transferred to the low-pressure gas-liquid separator 6 connected to the high-pressure gas-liquid separator 5 disposed at the 1 m point of the upper part of the reaction tower, and the dynamic liquid phase holdup amount and dissolved hydrogen concentration were then measured.

The dynamic liquid phase holdup amount per unit catalyst exterior surface area was $0.10 \times 10^{-3}$ $m^3/m^2$. The dissolved hydrogen concentration was 0.7 kmol/$m^3$.

Subsequently, 500 ml of crude reaction product liquid phase thus obtained was placed in a 1,000 ml batch autoclave provided separately, and hydrogen gas was supplied with stirring, and the temperature was raised to 220°C, and the saturated hydrogen concentration at 18 MPa was then measured, and was found to be 1.5 kmol/$m^3$. That is to say, the dissolved hydrogen concentration at the 1 m point of the upper part of the reaction tower was 47% of the saturated hydrogen concentration in the liquid phase.

The strength of the charged catalyst was weakest in the transverse direction, and the mean value A of the minimum crushing strength was 6.1 kg and σ was 2.0, and thus (A-2σ) was 2.1kg. The solid catalyst was not disintegrated after 500 hours' operation, nor was any change involving pressure loss observed.

Example 3 (inventive)

Fatty acid (iodine value 57.0, glc composition C12: 0.1%, C14: 2.2%, C16F0: 22.6%, C16F1: 4.8%, C18F0: 13.7%, C18F1: 46.3%, C18F2: 5.1%, C18F3: 0.6%, C20F2: 0.4%, the sum of $C_{15}$, $C_{17}$, and $C_{19}$ fatty acids: 4.2%), obtained by hydrolysis of beef tallow/lard mixed fats and oils, was esterified with methyl alcohol using p-toluenesulfonic acid, and the methyl ester product after having been washed with water was used as a starting material (sulfur content of 2.5 ppm, chlorine content of 1.0 ppm).

Operation was conducted continuously under trickle flow condition for 500 hours in the same manner as in Example 1 except that the starting material feed rate per square meter of the cross sectional area of the reaction tower was set to 10 $m^3$/h and the hydrogen gas rate per square meter of the cross sectional area of the reaction tower was set to 1,000 $m^3$/h, whereby a beef tallow and lard mixed alcohol crude product was obtained. The reaction results are shown in Table 3.

Table 3

| | Saponification value conversion (mol%) | Iodine value of reaction product (100 g$I_2$/g) |
|---|---|---|
| after 100 hours | 99.0 | 0.1 |
| after 300 hours | 99.0 | 0.1 |
| after 500 hours | 98.6 | 0.1 |

It was found that a steady state had been attained 10 hours after the start of the reaction. At that time, a crude reaction product liquid phase was withdrawn in a predetermined amount and transferred to the low-pressure gas-liquid separator 6 connected to the high-pressure gas-liquid separator 5 disposed at the 1 m point of the upper part of the reaction tower, and the dynamic liquid phase holdup amount and dissolved hydrogen concentration were then measured.

The dynamic liquid phase holdup amount per unit catalyst exterior surface area was $0.08 \times 10^{-3}$ $m^3/m^2$. The dissolved hydrogen concentration was 1.1 $kmol/m^3$

Subsequently, 500 ml of crude reaction product in the liquid phase thus obtained was placed in a 1,000 ml batch autoclave provided separately, and hydrogen gas was supplied with stirring, the temperature was raised to 220°C, and the saturated hydrogen concentration at 18 MPa was then measured, and was found to be 1.5 $kmol/m^3$ That is to say, the dissolved hydrogen concentration at the 1 m of the upper part of the reaction tower was 73% of the saturated hydrogen concentration in the liquid phase.

The strength of the charged catalyst was weakest in the transverse direction, and the mean value A of the minimum crushing strength was 6.1 kg and σ was 2.0, and thus (A-2σ) was 2.1kg. The solid catalyst was not disintegrated after 500 hours' operation, nor was any change involving pressure loss observed.

Example 4 (inventive)

Used vegetable oil (iodine value 119.5, glc composition C14: 0.4%, C16: 12.0%, C16F1: 0.7%, C18: 4.3%, C18F1: 40.8%, C18F2: 34.1%, C18F3: 7.3%, C20F1: 0.4%) was subjected to transesterification with methyl alcohol using sodium hydroxide, and the reaction mixture was neutralized with 10% hydrochloric acid, and washed with water, giving methyl ester product (sulfur content of 3.3 ppm, chlorine content of 4.2 ppm). The methyl ester product obtained was used as a starting material.

Operation was continuously conducted for 500 hours under trickle flow condition in the same manner as in Example 1 with the exception of using said starting material, whereby a reduced vegetable oil-derived alcohol crude product was produced.

The reaction results are shown in Table 4 below. As seen from Table 4, catalytic activity reduces over long term operation, since sulfur content and chlorine content are high. At the early stages of the reaction, however, a good result was obtained.

Table 4

|  | Saponification value conversion (mol%) |
|---|---|
| after 100 hours | 99.0 |
| after 300 hours | 89.4 |
| after 500 hours | 68.6 |

It was found that a steady state had been attained 10 hours after the start of the reaction. At that time, a crude reaction product liquid phase was withdrawn in a predetermined amount and transferred to the low-pressure gas-liquid separator 6 connected to the high-pressure gas-liquid separator 5 disposed at the 1 m point of the upper part of the reaction tower, and the dynamic liquid phase holdup amount and dissolved hydrogen concentration were then measured.

The dynamic liquid phase holdup amount per unit catalyst exterior surface area was $0.09 \times 10^{-3}$ $m^3/m^2$. The dissolved hydrogen concentration was 0.8 $kmol/m^3$.

Subsequently, 500 ml of the crude reaction product liquid phase thus obtained was placed in a 1,000 ml batch autoclave provided separately, hydrogen gas was supplied with stirring, and the temperature was raised to 220°C, and the saturated hydrogen concentration at 18 MPa was then measured, and found to be 1.5 $kmol/m^3$. That is to say, the dissolved hydrogen concentration at the 1 m point of the upper part of the reaction tower was 53% of the saturated hydrogen concentration in the liquid phase.

The strength of the charged catalyst was weakest in the transverse direction, and the mean value A of the minimum crushing strength was 6.1 kg and σ was 2.0, and thus (A-2σ) was 2.1kg. Disintegration of the solid catalyst after 500 hours' operation was not observed, nor was any change involving pressure loss observed.

Example 5: Hydrogenation to saturated alcohol (Reference)

Each reactor was charged with a tablet-shaped copper oxide-calcium silicate catalyst (bulk specific gravity of 1.5 kg/l, diameter of 3 mm, height of 3 mm) manufactured with a tabletting machine.

Palm kernel oil was transesterified with methyl alcohol using sodium hydroxide, and the product was then distilled, giving saturated fatty acid methyl esters (glc composition: C12: 75%, C14: 25%) (sulfur content: 0.2 ppm, chlorine content: 0.5 ppm). Operation was conducted continuously under trickle flow condition for 500 hours in the same manner as in Example 1 except that the starting material feed rate per square meter of the cross section of the reaction tower was set to 15 m³/h, the hydrogen gas rate per square meter of the cross section of the reaction tower was set to 800 m³/h, the temperature was set to 190°C and the pressure was set to 20 Mpa, whereby fractionated palm kernel oil-derived alcohol crude product was obtained as the hydrogenation product. The reaction results are shown in Table 5.

Table 5

|  | Saponification value conversion (mol%) |
|---|---|
| after 100 hours | 99.2 |
| after 300 hours | 99.2 |
| after 500 hours | 99.2 |

It was found that a steady state had been attained 10 hours after the start of the reaction. At that time, a crude reaction product liquid phase was withdrawn in a predetermined amount and transferred to the low-pressure gas-liquid separator 6 connected to the high-pressure gas-liquid separator 5 at the 1 m point of the upper part of the reactor, and the dynamic liquid phase holdup amount and dissolved hydrogen concentration were then measured.

The dynamic liquid phase holdup amount per unit catalyst exterior surface area was $0.09 \times 10^{-3}$ m³/m². The dissolved hydrogen concentration was 0.8 kmol/m³.

Subsequently, 500 ml of the crude reaction product liquid phase thus obtained was placed in a 1,000 ml batch autoclave provided separately, hydrogen gas was supplied with stirring, the temperature was raised to 190°C and the saturated hydrogen concentration at 20 MPa was measured, and found to be 1.6 kmol/m³. That is to say, the dissolved hydrogen concentration at the 1 m point of the upper part of the reaction tower was 50% of the saturated hydrogen concentration in the liquid phase.

The strength of the charged catalyst was weakest in the transverse direction, and the mean value A of the minimum crushing strength was 10.3 kg and σ was 3.5, and thus (A-2σ) was 3.3 kg. Disintegration of the solid catalyst after 500 hours' operation was not observed, nor was any change involving pressure loss observed.

Example 6 : Hydrogenation to saturated alcohol (Reference)

Each reactor was charged with a tablet-shaped copper-zinc-aluminum oxide catalyst (bulk specific gravity of 1.6 kg/l, diameter of 3 mm $\times$ height of 3 mm) manufactured with a tabletting machine.

Palm kernel oil was transesterified with methyl alcohol using sodium hydroxide, and the product was then distilled, giving saturated fatty acid methyl esters (glc composition: C12: 75%, C14: 25%) (sulfur content: 0.2 ppm, chlorine content: 0.5 ppm). Operation was conducted continuously under trickle flow condition for 500 hours in the same manner as in Example 1 except that the starting-material feed rate per square meter of the cross sectional area of the reaction tower was set to 15 m³/h, the hydrogen gas rate per square meter of the cross sectional area of the reaction tower was set to 800 m³/h, the temperature was set to 230°C and the pressure was set to 20 Mpa, whereby fractionated palm kernel oil-derived alcohol crude product was obtained as a hydrogenation product. The reaction results are shown in Table 6.

Table 6

|  | Saponification value conversion (mol%) |
|---|---|
| after 100 hours | 99.0 |
| after 300 hours | 99.0 |
| after 500 hours | 99.0 |

It was found that a steady state had been attained 10 hours after the start of the reaction. At that time, a crude reaction product liquid phase was withdrawn in a predetermined amount and transferred to the low-pressure gas-liquid separator 6 connected to the high-pressure gas-liquid separator 5 disposed at the 1 m point of the upper part of the reaction tower, and the dynamic liquid phase holdup amount and dissolved hydrogen concentration were then measured.

The dynamic liquid phase holdup amount per unit catalyst exterior surface area was $0.09 \times 10^{-3}$ m³/m². The dissolved hydrogen concentration was 0.8 kmol/m³.

Subsequently, 500 ml of the crude reaction product liquid phase thus obtained was placed in a 1,000 ml batch autoclave provided separately, hydrogen gas was supplied with stirring, and the temperature was raised to 230°C and the saturated hydrogen concentration at 20 MPa was then measured, and found to be 1.6 kmol/m³. That is to say, the dissolved hydrogen concentration at the 1 m point of the upper part of the reaction tower was 50% of the saturated hydrogen concentration in the liquid phase.

The strength of the charged catalyst was weakest in the transverse direction, and the mean value A of the minimum crushing strength was 6.6 kg and σ was 2.5, and thus (A-2σ) was 1.6 kg. Disintegration of the solid catalyst after 500 hours' operation was not observed, nor was any change involving pressure loss observed.

Example 7: Hydrogenation of dicarboxylic acid diester to diol (Reference)

Reaction was conducted continuously under trickle flow condition for 500 hours in the same manner as in Example 1 except that sebacic acid dimethyl ester (sulfur content of 0.05 ppm, chlorine content of 0.05 ppm) was supplied, giving the corresponding diol, namely crude 1,10-decane diol. The reaction results are shown in Table 7. Table 7 shows that the reaction advanced without any problem, and produced excellent effects.

Table 7

|  | Saponification value conversion (mol%) |
| --- | --- |
| after 100 hours | 99.0 |
| after 300 hours | 99.0 |
| after 500 hours | 99.0 |

It was found that a steady state had been attained 10 hours after the start of the reaction. At that time, a crude reaction product liquid phase was withdrawn in a predetermined amount and transferred to the low-pressure gas-liquid separator 6 connected to the high-pressure gas-liquid separator 5 disposed at the 1 m point of the upper part of the reaction tower and the dynamic liquid phase holdup amount and dissolved hydrogen concentration were then measured.

The dynamic liquid phase holdup amount per unit catalyst exterior surface area was $0.09 \times 10^{-3}$ m$^3$/m$^2$. The dissolved hydrogen concentration was 0.6 kmol/m$^3$.

Subsequently, 500 ml of the crude reaction product liquid phase thus obtained was placed in a 1,000 ml batch autoclave provided separately, hydrogen gas was supplied with stirring, the temperature was raised to 220°C, and the saturated hydrogen concentration at 18 MPa was measured, and found to be 1.4 kmol/m$^3$. That is to say, the dissolved hydrogen concentration at the 1 m point of the upper part of the reaction tower was 43% of the saturated hydrogen concentration in the liquid phase.

The strength of the charged catalyst was weakest in the transverse direction, and the mean value A of the minimum crushing strength was 6.1 kg and σ was 2.0, and thus (A-2σ) was 2.1 kg. Disintegration of the solid catalyst after 500 hours' operation was not observed, nor was any change involving pressure loss observed.

Example 8 (inventive)

Reactors 1 to 4 were charged with a cylindrical zinc-chromium oxide catalyst (bulk specific gravity of 1.4 kg/l, diameter of 5 mm, height of 5 mm) manufactured with a tabletting machine.

Fatty acids obtained by hydrolysis of palm kernel oil was distilled, and the product was subjected to a fractionated solidification by cooling, giving unsaturated fatty acids (iodine value 93.4, glc composition: C12: 0.6%, C14: 0.6%, C16: 5.5%, C18: 1.4%, C18F1: 78.5%, C18F2: 11.8%, C18F3: 0.5%, C20F2: 0.3%) (trade name: "PALMAC 750", manufactured by Acidchem). The obtained unsaturated fatty acids were esterified with methyl alcohol in the absence of a catalyst, giving a methyl ester product. The methyl ester product (sulfur content of 0.05 ppm, chlorine content of 0.05 ppm) was used as a starting material.

Reaction was carried out using said starting material under trickle flow condition for 500 hours at a starting material feed rate per square meter of the cross sectional area of the reaction tower of 2.1 m$^3$/h, a hydrogen gas feed rate per square meter of the cross sectional area of the reaction tower of 80 m$^3$/h, a temperature of 270°C, and pressure of 25 Mpa, giving crude unsaturated palm kernel oil alcohols as the hydrogenation product. The reaction results are shown in Table 8.

Table 8

|  | Saponification value conversion (mol%) | Iodine value of reaction product (100 gI$_2$/g) |
| --- | --- | --- |
| after 100 hours | 94.6 | 93.1 |
| after 300 hours | 94.5 | 93.2 |
| after 500 hours | 94.6 | 93.1 |

It was found that a steady state had been attained 10 hours after the start of the reaction. At that time, a crude reaction product liquid phase was withdrawn in a predetermined amount and transferred to the low-pressure gas-liquid separator 6 connected to the high-pressure gas-liquid separator 5 disposed at the 1 m point of the upper part of the reaction tower, and the dynamic liquid phase holdup amount and dissolved hydrogen concentration were then measured.

The dynamic liquid phase holdup amount per unit catalyst exterior surface area was $0.07 \times 10^{-3}$ $m^3/m^2$. The dissolved hydrogen concentration was 1.5 $kmol/m^3$.

Subsequently, 500 ml of the crude reaction product liquid phase thus obtained was placed in a 1,000 ml batch autoclave provided separately, hydrogen gas was supplied with stirring, the temperature was raised to 270°C, and the saturated hydrogen concentration at 25 MPa was measured, and found to be 1.5 $kmol/m^3$. That is to say, the dissolved hydrogen concentration at the 1 m point of the upper part of the reaction tower was 100% of the saturated hydrogen concentration in the liquid phase.

The strength of the charged catalyst was weakest in the transverse direction, and the mean value A of the minimum crushing strength was 9.4 kg and $\sigma$ was 3.2, and thus (A-2$\sigma$) was 3.0 kg. Disintegration of the solid catalyst after 500 hours' operation was not observed, nor was any change involving pressure loss observed.

Example 9 (Reference)

Reactors 1 to 4 were charged with a cylindrical 0.5 wt.% ruthenium-alumina catalyst (diameter of 3 mm, height of 3 mm) manufactured with a tabletting machine.

Reaction was carried out using a 50 wt.% diglyme solution of methylenedianiline (sulfur content: 0.01 ppm or less (detection limit or below), chlorine content: 0.01 ppm or less (detection limit or below)) as a starting material under trickle flow condition for 500 hours at a starting material feed rate per square meter of the cross sectional area of the reaction tower of 15 $m^3/h$, a hydrogen gas rate per square meter of the cross sectional area of the reaction tower of 60 $m^3/h$, a temperature of 130°C, and a pressure of 10 Mpa, giving crude bis(4-aminocyclohexyl)methane as a nuclear hydrogenation product. The reaction results are shown in Table 9.

Table 9

| Operation time | Nuclear hydrogenation GLC conversion (mol%) |
|---|---|
| after 100 hours | 99.99 |
| after 300 hours | 99.99 |
| after 500 hours | 99.99 |

It was found that a steady state had been attained 10 hours after the start of the reaction. At that time, a crude reaction product liquid phase was withdrawn in a predetermined amount and transferred to the low-pressure gas-liquid separator 6 connected to the high-pressure gas-liquid separator 5 disposed at the 1 m point of the upper part of the reaction tower, and the dynamic liquid phase holdup amount and dissolved hydrogen concentration were then measured.

The dynamic liquid phase holdup amount per unit catalyst exterior surface area was $0.09 \times 10^{-3}$ $m^3/m^2$. The dissolved hydrogen concentration was 0.5 $kmol/m^3$.

Subsequently, 500 ml of the crude reaction product liquid phase thus obtained was placed in a 1,000 ml batch autoclave provided separately, hydrogen gas was supplied with stirring, and a temperature was raised to 220°C and the saturated hydrogen concentration at 25 MPa was measured, and found to be 2.0 $kmol/m^3$. That is to say, the dissolved hydrogen concentration at the point of 1 m point of the upper part of the reaction tower was 25% of the saturated hydrogen concentration in the liquid phase.

The strength of the charged catalyst was weakest in the transverse direction, and the mean value A of the minimum crushing strength was 9.4 kg and $\sigma$ was 3.2, and thus (A-2$\sigma$) was 3.0 kg. Disintegration of the solid catalyst after 500 hours' operation was not observed, nor was any change involving pressure loss observed.

INDUSTRIAL APPLICABILITY

The present invention provides a method of carrying out, with long-term stability and high productivity, heterogeneous catalytic hydrogenation, in particular reduction reaction of unsaturated fatty acid alkyl esters to unsaturated alcohols.

**Claims**

1. A heterogeneous catalytic hydrogenation process comprising hydrogenating a substance to be hydrogenated under trickle flow condition using a reaction tower charged with a solid catalyst in which hydrogen gas and a liquid phase containing the substance to be hydrogenated are made to flow co-currently downward from above, wherein the dynamic liquid phase holdup amount per unit catalyst exterior surface area of the catalyst charged in the reaction tower is 0.005 X $10^{-3}$ to 0.14 X $10^{-3}$ m³/m² and the catalyst has a catalyst strength of 1.0 kg to 4.0 kg, as determined by the following formula

$$\text{catalyst strength} = A - 2\sigma$$

wherein A represents a mean value of minimum crushing strengths of 100 catalyst samples measured according to the method described in "Test method for crushing strength" of JIS Z 8841-1993 and σ represents a standard deviation,
the total content of chlorine atom and sulfur atom contained in the substance to be hydrogenated that is subjected to the hydrogenation is 5 ppm or less,
the solid catalyst is a zinc-based catalyst and the hydrogenation reaction is reduction of an unsaturated fatty acid alkyl ester to an unsaturated alcohol.

2. The hydrogenation process according to Claim 1, wherein
the hydrogenation reaction is reduction of a $C_{16}$-$C_{22}$ unsaturated fatty acid $C_1$-$C_4$ alkyl ester to a $C_{16}$-$C_{22}$ unsaturated alcohol.

3. The hydrogenation process according to any one of Claims 1-2, wherein the total content of chlorine atom and sulfur atom contained in the substance to be hydrogenated that is subjected to the hydrogenation is 3 ppm or less.

4. The hydrogenation process according to Claim 1, wherein the zinc-based catalyst is zinc-chromium oxide, zinc-aluminum oxide, zinc-aluminum-chromium oxide, zinc-chromium-manganese oxide, zinc-iron oxide or zinc-iron-aluminum oxide.

5. The hydrogenation process according to any one of Claims 1-4, wherein the solid catalyst has a shape selected from a cylinder, a hollow cylinder, a trilobal-shaped rod, a tetralobal-shaped rod, and a spherical shape, and has a minimum length of 1 to 10 mm.

6. The hydrogenation process according to any one of Claims 1-5, wherein the dissolved hydrogen concentration in the liquid phase present in the reaction tower is 0.01 to 5.0 kmol/m³.

7. The hydrogenation process according to Claim 1, wherein the dissolved hydrogen concentration in the liquid phase is maintained within 10 to 100% of saturated hydrogen concentration in the liquid phase at a point 1 m below the highest point of the catalyst layer in the reaction tower.

8. The hydrogenation process according to Claim 1, wherein the hydrogenation reaction is hydrogenation of an unsaturated carboxylic acid ester to an unsaturated alcohol, and the dissolved hydrogen concentration in the liquid phase is maintained within 50 to 100% of the saturated hydrogen concentration in the liquid phase, at a point 1 m below the highest point of the catalyst layer in the reaction tower.


**Patentansprüche**

1. Heterogenes katalytisches Hydrierungsverfahren, umfassend die Hydrierung einer zu hydrierenden Substanz unter Rieselströmungsbedingung unter Verwendung eines mit einem festen Katalysator beladenen Reaktionsturms, in dem man Wasserstoffgas und eine flüssige Phase, die die zu hydrierende Substanz enthält, im Gleichstrom von oben nach unten strömen lässt, wobei die dynamische Flüssigphasen-Holdup-Menge pro äußerer Katalysatoroberflächeneinheit des in dem Reaktionsturm gefüllten Katalysators 0,005 × $10^{-3}$ bis 0,14 × $10^{-3}$ m³/m² beträgt und der Katalysator eine Katalysatorfestigkeit von 1,0 kg bis 4,0 kg aufweist, bestimmt durch die folgende Formel

$$\text{Katalysatorfestigkeit} = A\text{-}2\sigma$$

worin A einen Mittelwert der Mindestbruchfestigkeiten von 100 Katalysatorproben, gemessen nach dem Verfahren, das in "Prüfverfahren zur Bruchfestigkeit" von JIS Z 8841-1993 beschrieben ist, darstellt und $\sigma$ eine Standardabweichung darstellt,

wobei der Gesamtgehalt an Chloratom und Schwefelatom, die in der zu hydrierenden Substanz, die der Hydrierung unterworfen wird, enthalten sind, 5 ppm oder weniger beträgt,

der feste Katalysator ein Zink-basierter Katalysator ist und die Hydrierungsreaktion die Reduktion eines ungesättigten Fettsäurealkylesters zu einem ungesättigten Alkohol ist.

2. Hydrierungsverfahren nach Anspruch 1, wobei

die Hydrierungsreaktion die Reduktion eines ungesättigten $C_{16}$-$C_{22}$-Fettsäure-$C_1$-$C_4$-alkylesters zu einem ungesättigten $C_{16}$-$C_{22}$-Alkohol ist.

3. Hydrierungsverfahren nach irgendeinem der Ansprüche 1-2, wobei der Gesamtgehalt an Chloratom und Schwefelatom, die in der zu hydrierenden Substanz, die der Hydrierung unterworfen wird, enthalten sind, 3 ppm oder weniger beträgt.

4. Hydrierungsverfahren nach Anspruch 1, wobei der Zink-basierte Katalysator Zink-Chrom-Oxid, Zink-Aluminium-Oxid, Zink-Aluminium-Chrom-Oxid, Zink-Chrom-Mangan-Oxid, Zink-Eisen-Oxid oder Zink-Eisen-Aluminium-Oxid ist.

5. Hydrierungsverfahren nach irgendeinem der Ansprüche 1-4, wobei der feste Katalysator eine Form ausgewählt aus einem Zylinder, einem Hohlzylinder, einem trilobalförmigen Stab, einem tetralobalförmigen Stab und einer kugelförmigen Form aufweist und eine Mindestlänge von 1 bis 10 mm aufweist.

6. Hydrierungsverfahren nach irgendeinem der Ansprüche 1-5, wobei die gelöste Wasserstoffkonzentration in der im Reaktionsturm vorhandenen flüssigen Phase 0,01 bis 5,0 kmol/m$^3$ beträgt.

7. Hydrierungsverfahren nach Anspruch 1, wobei die gelöste Wasserstoffkonzentration in der flüssigen Phase innerhalb von 10 bis 100% der gesättigten Wasserstoffkonzentration in der flüssigen Phase an einem Punkt 1 m unterhalb des höchsten Punktes der Katalysatorschicht in dem Reaktionsturm gehalten wird.

8. Hydrierungsverfahren nach Anspruch 1, wobei die Hydrierungsreaktion die Hydrierung von einem ungesättigten Carbonsäureester zu einem ungesättigten Alkohol ist und die gelöste Wasserstoffkonzentration in der flüssigen Phase innerhalb von 50 bis 100% der gesättigten Wasserstoffkonzentration in der flüssigen Phase an einem Punkt 1 m unterhalb des höchsten Punktes der Katalysatorschicht in dem Reaktionsturm gehalten wird.

**Revendications**

1. Procédé d'hydrogénation catalytique hétérogène comprenant l'hydrogénation d'une substance à hydrogéner dans des conditions d'écoulement ruisselant en utilisant une tour de réaction chargée avec un catalyseur solide dans laquelle l'hydrogène gazeux et une phase liquide contenant la substance à hydrogéner sont amenés à s'écouler dans un courant conjoint en aval du dessus, dans lequel la quantité de conservation de phase liquide dynamique par unité d'aire surfacique extérieure de catalyseur du catalyseur chargé dans la tour de réaction est de 0,005 x 10$^{-3}$ à 0,14 x 10$^{-3}$ m$^3$/m$^2$ et le catalyseur a une résistance de catalyseur de 1,0 kg à 4,0 kg, telle que déterminée par la formule suivant :

$$\text{résistance de catalyseur} = A\text{--}2\sigma$$

dans laquelle A représente une valeur moyenne des résistances au broyage minimum de 100 échantillons de catalyseur mesurée selon la méthode décrite dans « Méthode de test pour la résistance au broyage » de la norme JIS Z 8841-1993 et $\sigma$ représente un écart type,

la teneur totale en atome de chlore et en atome de soufre contenue dans la substance à hydrogéner qui est soumise à l'hydrogénation est de 5 ppm ou moins,
le catalyseur solide est un catalyseur à base de zinc et la réaction d'hydrogénation est la réduction d'un ester d'alkyle d'acide gras insaturé en un alcool insaturé.

2. Procédé d'hydrogénation selon la revendication 1, dans lequel la réaction d'hydrogénation est la réduction d'un ester d'alkyle en $C_1$ à $C_4$ d'acide gras insaturé en $C_{16}$ à $C_{22}$ en un alcool insaturé en $C_{16}$ à $C_{22}$.

3. Procédé d'hydrogénation selon l'une quelconque des revendications 1 à 2, dans lequel la teneur totale en atome de chlore et en atome de soufre contenue dans la substance à hydrogéner qui est soumise à l'hydrogénation est de 3 ppm ou moins.

4. Procédé d'hydrogénation selon la revendication 1, dans lequel le catalyseur à base de zinc est un oxyde de zinc-chrome, un oxyde de zinc-aluminium, un oxyde de zinc-aluminium-chrome, un oxyde de zinc-chrome-manganèse, un oxyde de zinc-fer ou un oxyde de zinc-fer-aluminium.

5. Procédé d'hydrogénation selon l'une quelconque des revendications 1 à 4, dans lequel le catalyseur solide a une forme choisie parmi un cylindre, un cylindre creux, une tige de forme trilobée, une tige de forme tétralobée, et une forme sphérique et a une longueur minimale de 1 à 10 mm.

6. Procédé d'hydrogénation selon l'une quelconque des revendications 1 à 5, dans lequel la concentration en hydrogène dissous dans la phase liquide présente dans la tour de réaction est de 0,01 à 5,0 kmol/m$^3$.

7. Procédé d'hydrogénation selon la revendication 1, dans lequel la concentration en hydrogène dissous dans la phase liquide est maintenue dans les 10 à 100 % de la concentration en hydrogène saturée dans la phase liquide à un point situé 1 m en dessous du point le plus élevé de la couche de catalyseur dans la tour de réaction.

8. Procédé d'hydrogénation selon la revendication 1, dans lequel la réaction d'hydrogénation est l'hydrogénation d'un ester d'acide carboxylique insaturé en un alcool insaturé et la concentration en hydrogène dissous dans la phase liquide est maintenue dans les 50 à 100 % de la concentration en hydrogène saturée dans la phase liquide à un point situé 1 m en dessous du point le plus élevé de la couche de catalyseur dans la tour de réaction.

Fig. 1

Fig. 2

Fig. 3

Fig. 4

**REFERENCES CITED IN THE DESCRIPTION**

**Patent documents cited in the description**

- JP 2544552 B
- JP 2846450 B
- JP 2934073 B
- JP 2001089403 A

**Non-patent literature cited in the description**

- *Chemical Engineering,* 1970, vol. 34 (12), 1260